# EUROPEAN PATENT SPECIFICATION

(11) **EP 3 866 817 B1**
(45) Date of publication and mention of the grant of the patent: **02.12.2026**
(21) Application number: 19874286.8
(22) Date of filing: 17.10.2019
(51) Int. Cl.: A61K 35/32, A61F 2/30, A61L 27/36, A61F 2/28, A61F 2/46

(54) **MAMMAL-DERIVED MATRIX AS A SAFE BONE REPLACEMENT**
AUS SÄUGETIEREN GEWONNENE MATRIX ALS SICHERER KNOCHENERSATZ
MATRICE DÉRIVÉE D'UN MAMMIFÈRE EN TANT QUE SUBSTITUT OSSEUX SÛR

(30) Priority: 18.10.2018 IL 26247318
(43) Date of publication of application: 25.08.2021
(73) Proprietor: Ivory Graft Ltd., 5885849 Holon (IL)
(72) Inventor: SAPOZNIKOV, Larion, 5246105 Ramat Gan (IL); SAHAR, Meir, 5846401 Holon (IL); TSATURYAN, Ruben, Moscow (RU)
(74) Representative: Maslanka Kubik, Dorota Irena
(86) International application number: PCT/IL2019/051125
(87) International publication number: WO 2020/079690

(56) References cited:
- EP-A1- 2 692 364
- WO-A1-2014/111925
- WO-A1-2018/169442
- US-A1- 2013 220 365
- HORTON, M. A. ET AL.: "Arg Gly Asp (RGD) peptides and the anti-vitronectin receptor antibody 23C6 inhibit dentine resorption and cell spreading by osteoclasts", EXPERIMENTAL CELL RESEARCH, vol. 195, no. 2, 1 September 1991 (1991-09-01), pages 368 - 375, XP002703707, DOI: 10.1016/0014-4827(91)90386-9

## Description

### Field of the Invention

The present invention relates to a method of manufacturing a matrix for using as bone graft or bone replacement. Particularly, mammalian teeth or mammalian bone is processed to provide a safe xenograft in regenerative medicine. Document WO 2014/111925 A1 is the closest prior art and discloses methods involving mammalian tissues, but does not specifically address the distinct requirements and challenges associated with preparing xenografts.

### Background of the Invention

Bone grafting is a surgical procedure that replaces missing bone and is widely used in regenerative medicine, for example in dental surgery, in treating fractures, or after resection of bone tumors (see, for example, The Merck Manual, 19th Ed., 2011, p. 364). Bone can regenerate but needs a graft as a scaffold. The graft may originate from the patient's own body - autograft - or it can come from other donor or from cadaveric bone - allograft; the graft may be synthetic. The graft material should preferably be osteoconductive, i.e. it should support the growth of the new bone on its surface; the material should preferably be also osteoinductive, i.e. it should induce the process of osteogenesis. Since tooth dentine is very similar to bone, extracted teeth were considered for preparing a bone graft; for example EP 2 462 899 relates to an alveolar bone graft prepared from patient's own tooth, processes by dewatering in alcohol, degreasing in ether, and decalcification in hydrochloric acid. It would be highly desirable to use a teeth scaffold material for any subject in need and not only for the tooth donor as an autograft, but a danger of strong immune responses or viral and other infections restricts the use of such grafts in other subjects of the same species (allograft), and still more the use of such grafts in subjects of a different species (xenograft). US 9,610,383 attempts to overcome the problem by irradiating extracted teeth or bones by ultrasonic waves at a reduced pressure, followed by chemical treatments, including a treatment with hydrochloric acid. Still stricter safety regulations necessitate the development of new matrices for other than autograft applications. It is therefore an object of this invention to provide a matrix as a bone substitute for bone replacement applications.

It is another object of this invention to provide a method for producing a scaffold matrix for bone grafting, based on mammalian teeth and applicable instead of allograft.

It is still another object of this invention to provide a method for producing a scaffold matrix for bone grafting, based on mammalian teeth and applicable as xenograft.

It is a further object of this invention to provide a matrix based on mammalian teeth for safe use as a bone substitute in both allograft and xenograft applications.

Other objects and advantages of present invention will appear as description proceeds.

### Summary of the Invention

The present invention provides a xenograft bone graft or xenograft bone replacement material as defined in claim 1.

The invention relates to a method of manufacturing xenograft bone graft or bone replacement material as defined in claim 2.

In a perferred embodiment of the invention, said method comprises the steps of immersing said crushed mammalian tooth or bone or a part thereof in an alcoholic solution of an alkali hydroxide for a period of from 0.25 to 5 hour; washing said crushed material after said immersing step with a saline solution; and heating said crushed and washed material at a temperature of between 110 and 190°C for a period of from 0.1 to 3 hour.

In a preferred embodiment of the invention, said part of the tooth comprises dentine. In one preferred embodiment of the invention, the mammalian teeth are processed to separate fractions containing dentine for further steps. The method of the invention may comprise a step of removing soft tissue before further processing, or removing enamel before further processing, or removing both and performing steps ii) to iv) without these components. In a preferred embodiment of the invention, the process further comprises γ-irradiation. Said alcohol comprises lower alcohol, preferably ethanol. Said alkali hydroxide may comprise sodium hydroxide.

Said alcohol is preferably aqueous ethanol, 50-90 vol. %, such as about 70% vol., and said hydroxide is sodium hydroxide in a concentration of up to saturation in said aqueous ethanol at ambient temperature. Said step of immersing is performed preferably between 0.3 and 3 hour, such as between 0.5 and 1 hour, and said step of heating for from 0.2 to 2 hours. Said step of washing may comprise a saline solution, for example 0.15 M NaCl, possibly buffered. The washing may take from 0.75 to 1.5 hours, such as 1 hour. Said step of heating may take between 0.3 and 1 hours at 150°C, such as about 0.5 hours.

### Detailed Description of the Invention

It was shown that bone morphogenesis is induced by special peptides, found, for example, in demineralized bone or teeth (see, for example, US 6,677,306). Accordingly, many works attempted to provide bone grafts derived from demineralized bones or teeth, or from materials treated with hydrochloric acid (see already cited EP 2 462 899 and US 9,610,383). It has now been found that a powder obtained from mammalian teeth or parts thereof, comprising dentine and rid of cells, can advantageously serve as a safe bone graft if said teeth or parts thereof are treated with alcoholic hydroxide and heated to a temperature of 110°C or more, such as 150°C; it may seem surprising, as the instant conditions do not demineralize the teeth and, moreover, the conditions would supposedly destroy and/or remove the special growth peptides. The inventors believe, without wishing to be limited by any particular theory, that the instant teeth-derived material comprises residual dentine, which supports the bone regeneration. Particularly, the inventors believe that the teeth-derived material contains hydroxyapatite and residual collagen, which collagen, even if being modified by alcoholic hydroxide and by thermal treatment, exhibits osteoinductive effects and together with the hydroxyapatite also osteoconductive effects, the effects resulting from combined structural properties of the modified collagen protein and the inorganic matrix. According to the invention, the teeth-derived material provides a positive reaction with antibodies to the Arg-Gly-Asp (RGD) sequence; the RGD sequence is known to be comprised in receptors for cell adhesion molecules, as well as in collagen I which is a part of dentine.

Based on the above findings, the present invention is directed to a composition for treating bone fractures, or for replacing a missing bone in dental surgery, or for providing bone after resection of bone tumors. The composition has no toxicity, low immunogenicity, and is free of infectious agents. Particularly, the composition is absolutely free of any infectious or toxic agents, including eukaryotic microorganisms, spores, bacteria, viruses, proteins, lipopolysaccharides, or nucleic acids.

The composition of the invention comprises teeth-derived dentine or bone-derived base treated with alcoholic hydroxide and heated to a temperature of from 110 to 190°C, for example at about 150°C. In one embodiment, the teeth-derived or bone-derived material is heated at two steps at two different temperatures. The invention provides a composition to be used as a bone graft, derived from mammalian teeth or bones or their parts, for example from teeth rid of enamel and cellular components, exposed to an alkali hydroxide, such as sodium or potassium hydroxide in alcoholic solution, the solution comprising a lower alcohol, optionally mixed with another organic solvent or water. Said alcohol comprises, for example, ethanol. According to the invention, the composition positively reacts with anti-RGD antibodies.

The invention will be further described and illustrated in the following examples.

### Example

All steps were done in clean hood until blisters packaging stage. The process included grinding the teeth, for example pig teeth, and screening on 300 µm, and re-grinding the greater particles. The teeth were immersed for 40 minutes in sodium hydroxide dissolved in 70 vol% ethanol, 70 wt% NaOH + 30 wt% ethanol (70 vol%), while occasionally stirring to release non-dentin material and let it float and removing said floating material with a peristaltic pump. The alcoholic hydroxide was removed, and the crushed teeth were washed with distilled water ten times and allowed to be immersed in PBS for five minutes. The PBS was removed, and the immersion was repeated until the pH of the wash liquid was between 7.5 and 8.5. The crushed and washed teeth were placed on a stainless steel tray, a layer not higher than 1 cm, and heated in an oven for 10 minutes at 180°C and for 90 minutes at 130°C. If the loss on drying was higher than 5%, the heating at 130°C was prolonged by 30 minutes.

Desired portions were packed into glass or plastic vials or other medical class materials. If desired, portions were transferred to gamma radiation. No microbial growth was observed in the final package. Bio compatibility testing and the cytotoxicity in vitro tests were performed, and the product complied with the requirements.

While this invention has been described in terms of some specific examples, many modifications and variations are possible. It is therefore understood that within the scope of the appended claims, the invention may be realized otherwise than as specifically described.

## Claims

1. A xenograft bone graft or xenograft bone replacement material comprising a powder of crushed mammalian teeth, wherein the powder:
a) is acellular; and
b) comprises an arginylglycylaspartic acid, also known as RGD, peptide sequence, that is associated with collagen type I present in dentine and exhibits a positive reaction with an anti-RGD antibody.

2. A method of manufacturing a xenograft bone graft or xenograft bone replacement material of claim 1. comprising the steps of
i) providing a mammalian tooth or bone or a part thereof;
ii) immersing said mammalian tooth or bone or a part thereof in an alcoholic solution of an alkali hydroxide;
iii) washing said tooth or bone or a part thereof after immersing in said alcoholic hydroxide with a saline solution;
iv) heating and drying said washed tooth or bone or a part thereof at a temperature of between 110 and 190°C; and
v) crushing said tooth or bone or a part thereof, wherein the step of crushing is performed after any one of steps i) to iv);
said method further comprising γ-irradiation;
thereby providing an acellular powder of crushed mammalian teeth or parts thereof.

3. The method of claim 2, comprising the steps of immersing said crushed mammalian tooth or bone or a part thereof in the alcoholic solution of the alkali hydroxide for a period of from 0.25 to 5 hours; washing said crushed material after said immersing step with the saline solution; and heating said crushed and washed material for a period of 0.1 to 3 hours, wherein the temperature is between 120 and 180°C.

4. The method of claim 2, wherein said part of the tooth comprises dentine.

5. The method of claim 2, further comprising a step of removing soft tissue or enamel after step i) and performing steps ii) to iv) without these components.

6. The method of claim 2, wherein said alcohol comprises ethanol.

7. The method of claim 2, wherein said alkali hydroxide comprises sodium hydroxide.

8. The method of claim 2, wherein said alcohol is aqueous ethanol, 50-90 vol. %, and said hydroxide is sodium hydroxide in a concentration of up to saturation in said aqueous ethanol at ambient temperature.

9. The method of claim 2, wherein said immersing is performed for from 0.3 to 3 hours, and said step of heating for from 0.2 to 1 hour at 150°.

10. A xenograft bone graft or xenograft bone replacement of claim 1 configured for use in replacing a missing bone in dental surgery, configured for use in replacing missing bone in treating fractures, or configured for use in replacing missing bone after resection of bone tumors.

11. A matrix comprising mammalian teeth or bone or parts thereof treated with an alcoholic hydroxide and heated to a temperature of between 110 and 190°C according to the method of any of claims 2 to 9 for use in the preparation of a bone xenograft.

## Patentansprüche

1. Xenogenes Knochentransplantat- oder xenogenes Knochenersatzmaterial umfassend ein Pulver aus zerkleinerten Säugetierzähnen, wobei das Pulver:
a) azellulär ist; und
b) eine Peptidsequenz von Arginylglycylasparaginsäure, auch als RGD bekannt, umfasst, welche mit im Dentin vorhandenem Kollagen Typ I assoziiert ist und eine positive Reaktion mit einem Anti-RGD Antikörper aufweist.

2. Verfahren zur Herstellung eines xenogenen Knochentransplantat- oder xenogenen Knochenersatzmaterials nach Anspruch 1, umfassend die folgenden Schritte:
i) das Bereitstellen eines Säugetierzahns oder -knochens oder eines Teils derselben;
ii) das Eintauchen des genannten Säugetierzahns oder -knochens oder eines Teils derselben in eine alkoholische Lösung eines Alkalihydroxids;
iii) das Waschen des genannten Zahns oder Knochens oder eines Teils derselben nach dem Eintauchen in das genannte alkoholische Hydroxid mit einer Kochsalzlösung;
iv) das Erwärmen und das Trocknen des genannten gewaschenen Zahns oder Knochens oder eines Teils derselben bei einer Temperatur von zwischen 110 und 190 °C; und
v) das Zerkleinern des genannten Zahns oder Knochens oder eines Teils derselben, wobei der Zerkleinerungsschritt nach einem der Schritte i) bis iv) durchgeführt wird;
wobei das genannte Verfahren zusätzlich γ-Bestrahlung umfasst;
wodurch ein azelluläres Pulver von zerkleinerten Säugetierzähnen oder Teilen derselben bereitgestellt wird.

3. Verfahren nach Anspruch 2, umfassend die folgenden Schritte:
das Eintauchen des genannten zerkleinerten Säugetierzahns oder -knochens oder eines Teils derselben in die alkoholische Lösung des Alkalihydroxids eine Zeitdauer von 0,25 bis 5 Stunden lang;
das Waschen des genannten zerkleinerten Materials nach dem genannten Eintauchschritt mit der Kochsalzlösung; und
das Erwärmen des genannten zerkleinerten und gewaschenen Materials eine Zeitdauer von 0,1 bis 3 Stunden lang, wobei die Temperatur zwischen 120 und 180 °C liegt.

4. Verfahren nach Anspruch 2, wobei der genannte Teil des Zahns Dentin umfasst.

5. Verfahren nach Anspruch 2, zusätzlich umfassend einen Schritt des Beseitigens von Weichgewebe oder Zahnschmelz nach Schritt i) und das Durchführen der Schritte ii) bis iv) ohne diese Bestandteile.

6. Verfahren nach Anspruch 2, wobei der genannte Alkohol Ethanol umfasst.

7. Verfahren nach Anspruch 2, wobei das genannte Alkalihydroxid Natriumhydroxid umfasst.

8. Verfahren nach Anspruch 2, wobei der genannte Alkohol wässriges Ethanol, 50-90 Vol. %, ist und das genannte Hydroxid Natriumhydroxid in einer Konzentration von bis zur Sättigung im genannten wässrigen Ethanol bei Umgebungstemperatur ist.

9. Verfahren nach Anspruch 2, wobei das genannte Eintauchen von 0,3 bis 3 Stunden lang durchgeführt wird, und der genannte Erwärmungsschritt von 0,2 bis 1 Stunde lang bei 150 ° durchgeführt wird.

10. Xenogenes Knochentransplantat oder xenogener Knochenersatz nach Anspruch 1, welches/welcher für dessen Verwendung beim Ersatz eines fehlenden Knochens bei einer Zahnchirurgie ausgebildet ist, für dessen Verwendung beim Ersatz eines fehlenden Knochens bei der Behandlung von Brüchen ausgebildet ist oder für dessen Verwendung beim Ersatz eines fehlenden Knochens nach einer Resektion von Knochentumoren ausgebildet ist.

11. Matrix umfassend Säugetierzähne oder -knochen oder Teile derselben behandelt mit einem alkoholischen Hydroxid und erwärmt bis zu einer Temperatur von zwischen 110 und 190 °C gemäß dem Verfahren nach einem der Ansprüche 2 bis 9 für deren Verwendung bei der Zubereitung eines Knochenxenotransplantats.

## Revendications

1. Matériau de greffe osseuse de type xénogreffe ou de substitut osseux de type xénogreffe comprenant une poudre de dents de mammifère broyées, dans lequel la poudre :
a) est acellulaire ; et
b) elle comprend une séquence peptidique d'arginine-glycine-acide aspartique, également connue comme RGD, qui est associée au collagène de type 1 présent dans la dentine et montre une réaction positive avec un anticorps anti-RGD.

2. Procédé de fabrication d'un matériau de greffe osseuse de type xénogreffe ou substitut osseux de type xénogreffe selon la revendication 1, comprenant les étapes suivantes
i) fournir une dent ou un os de mammifère ou une partie de ceux-ci ;
ii) immerger ladite dent ou ledit os de mammifère ou une partie de ceux-ci dans une solution alcoolique d'un hydroxyde alcalin ;
iii) laver ladite dent ou ledit os ou une partie de ceux-ci après l'immersion dans ledit hydroxyde alcoolique avec une solution saline ;
iv) chauffer et sécher ladite dent ou ledit os ou une partie de ceux-ci lavé(e) à une température comprise entre 110 et 190 °C ; et
v) broyer ladite dent ou ledit os ou une partie de ceux-ci, dans lequel l'étape de broyage est réalisée après l'une quelconque des étapes i) à iv) ;
ledit procédé comprenant en outre une irradiation γ ;
fournissant ainsi une poudre acellulaire de dents de mammifère broyées ou de parties de celles-ci.

3. Procédé selon la revendication 2, comprenant les étapes d'immersion de ladite dent ou ledit os de mammifère ou une partie de ceux-ci broyé(e) dans la solution alcoolique de l'hydroxyde alcalin pendant une durée de 0,25 jusqu'à 5 heures ; lavage dudit matériau broyé après ladite étape d'immersion avec la solution saline ; et chauffage dudit matériau broyé et lavé pendant une durée de 0,1 jusqu'à 3 heures, dans lequel la température est comprise entre 120 et 180 °C.

4. Procédé selon la revendication 2, dans lequel ladite partie de la dent comprend de la dentine.

5. Procédé selon la revendication 2, comprenant en outre une étape d'élimination des tissus mous ou d'émail après l'étape i) et la réalisation des étapes ii) à iv) sans ces composants.

6. Procédé selon la revendication 2, dans lequel ledit alcool comprend de l'éthanol.

7. Procédé selon la revendication 2, dans lequel ledit hydroxyde alcalin comprend de l'hydroxyde de sodium.

8. Procédé selon la revendication 2, dans lequel ledit alcool est de l'éthanol aqueux, à 50-90 % en volume, et ledit hydroxyde est de l'hydroxyde de sodium dans une concentration jusqu'à saturation dans ledit éthanol aqueux à température ambiante.

9. Procédé selon la revendication 2, dans lequel ladite immersion est réalisée pendant 0,3 jusqu'à 3 heures, et ladite étape de chauffage est réalisée pendant 0,2 jusqu'à 1 heure à 150 °.

10. Greffe osseuse de type xénogreffe ou substitut osseux de type xénogreffe selon la revendication 1, configurée pour son utilisation dans la substitution d'un os manquant en chirurgie dentaire, configurée pour son utilisation dans la substitution d'un os manquant dans le traitement de fractures, ou configurée pour son utilisation dans la substitution d'un os manquant après la résection de tumeurs osseuses.

11. Matrice comprenant des dents ou des os de mammifère ou des parties de ceux-ci traités avec un hydroxyde alcoolique et chauffés jusqu'à une température comprise entre 110 et 190 °C selon le procédé selon l'une quelconque des revendications 2 à 9 pour son utilisation dans la préparation d'une xénogreffe osseuse.
